**Europäisches Patentamt**

**European Patent Office**

**Office européen des brevets**

(11) Veröffentlichungsnummer: **0 052 765**
**B1**

# (12) EUROPÄISCHE PATENTSCHRIFT

(45) Veröffentlichungstag der Patentschrift:
08.08.84

(21) Anmeldenummer: **81108632.1**

(22) Anmeldetag: **21.10.81**

(51) Int. Cl.³: **A 61 N 5/06**

(54) Vorrichtung zur phototherapeutischen Behandlung der Hyperbilirubinämie.

(30) Priorität: **24.11.80 DE 3044184**

(43) Veröffentlichungstag der Anmeldung:
**02.06.82 Patentblatt 82/22**

(45) Bekanntmachung des Hinweises auf die Patenterteilung:
**08.08.84 Patentblatt 84/32**

(84) Benannte Vertragsstaaten:
**AT BE CH DE FR GB IT LI NL SE**

(56) Entgegenhaltungen:
**FR - A - 2 193 628**
**FR - A - 2 342 745**
**FR - A - 2 389 229**
**US - A - 3 126 295**
**US - A - 3 658 068**

**WESTINGHOUSE ENGINEER, Band 31, Heft 5, September 1971, PITTSBURGH (US) "Special Blue Lamp Helps Treat Jaundice in Newborn Infants", Seiten 157-158**
**JOURNAL OF IES, Band 1, Heft 2, Januar 1972, NEW YORK (US) J.W. SAUSVILLE et al.: "Blue Lamps in Photography of Hyperbilirubinemia", Seiten 112-118**

(73) Patentinhaber: **Mutzhas, Maximilian F., Prof. Dr.,**
**Sonnenstrasse 17, D-8000 München 2 (DE)**

(72) Erfinder: **Mutzhas, Maximilian F., Prof. Dr.,**
**Sonnenstrasse 17, D-8000 München 2 (DE)**

(74) Vertreter: **Tetzner, Volkmar, Dr.-Ing. Dr. Jur.,**
**Van-Gogh-Strasse 3, D-8000 München 71 (DE)**

**Beschreibung**

Die Erfindung betrifft eine Vorrichtung zur phototherapeutischen Behandlung der Hyperbilirubinämie von Neugeborenen durch Bestrahlung.

Die bei Neugeborenen, insbesondere bei Frühgeburten, verhältnismäßig häufig auftretende Hyperbilirubinämie wird durch einen erhöhten Spiegel von Bilirubin, dem wesentlichsten Gallenfarbstoff ($C_{33}H_{33}O_6N_4$) hervorgerufen. Sie wird u. a. durch die noch nicht voll entwickelte Leberfunktion im Blutkreislauf von Neugeborenen verursacht. Durch diese verminderte Leberfunktion kann das fettlösliche Bilirubin nicht in wasserlösliche Produkte umgewandelt werden, die im Stuhl ausgeschieden werden können. Zusätzlich bewirkt sie eine erhöhte Durchlässigkeit der Blut-Hirnbarriere, so daß derart erkrankte Neugeborene anfälliger sind für Schäden im Zentralnervensystem, die durch Zerfallsprodukte des Bilirubins in den Hirnzellen entstehen können.

Bisher wird die Hyperbilirubinämie entweder chemotherapeutisch oder durch Phototherapie behandelt. Bei der phototherapeutischen Behandlung wird gemäß DIN 5031, Teil 10, Strahlung im Bereich von 405 nm bis 545 nm als wirksam angesehen, wobei das Maximum der Wirkungsfunktion etwa bei 450 bis 460 nm liegen soll. Für die Phototherapie finden bisher entweder Leuchtstofflampen mit blauem Leuchtstoff oder Lampen Verwendung, die einen großen Teil des sichtbaren Spektralbereiches emittieren und daher der Lichtfarbe Weiß zugeordnet werden können. Dabei erstreckt sich bei den blau leuchtenden Leuchtstofflampen das Spektrum etwa von 400 bis 550 nm, bei den weißen Leuchtstofflampen etwa von 300 bis 780 nm.

Weiterhin werden zur phototherapeutischen Behandlung der Hyperbilirubinämie Quecksilberdampf-Hochdruckstrahler verwendet, die mit einem zusätzlichen Außenkolben versehen sind, an dessen Innenseite sich ein Leuchtstoff befindet, der ebenfalls im Bereich von ca. 400 nm bis 550 nm stark emittiert.

Schließlich wird in technologisch weniger hoch entwickelten Gebieten teilweise auch noch die Heliotherapie betrieben, bei der die natürliche Sonne mit ihrem gesamten Spektrum als Strahlungsquelle dient.

Der Erfindung liegt nun die Aufgabe zugrunde, eine neue Vorrichtung zur phototherapeutischen Behandlung der Hyperbilirubinämie zu schaffen, die sich durch eine wesentlich verbesserte Wirksamkeit und zugleich durch die Vermeidung von mit den bekannten Bestrahlungseinrichtungen verbundenen Gefahren und Schäden auszeichnet.

Diese Aufgabe wird erfindungsgemäß dadurch gelöst, daß bei der neuen Vorrichtung zur phototherapeutischen Behandlung der Hyperbilirubinämie von Neugeborenen durch Bestrahlung höchstens 10% der im wirksamen Bereich von 405 bis 545 nm vorhandenen Strahlung unter 460 nm liegen.

Eingehende photobiologische Untersuchungen, die der Erfindung zugrunde liegen, haben nämlich gezeigt, daß — entgegen der bisherigen Auffassung der Fachwelt — sichtbares Licht aus dem blauen Spektralbereich (z. B. in Gegenwart von Chromophoren, wie Riboflavin) zu Zellschäden (DNA) führen kann, wobei das Maximum der spektralen Schädigungsfunktion etwa bei 450 nm liegt, also gerade etwa an der Stelle des Spektrums, an der die relative spektrale Wirkungsfunktion der Bilirubin-Dissoziation (nach DIN) ihr Maximum aufweist. Zellschäden (z. B. Mutagenese) sowie andere Schadwirkungen (z. B. phototoxischer Art) für die Haut bzw. Augenschäden (blue light hazard) können jedoch zum Teil gefährlicher sein als die durch das Bilirubin selbst hervorgerufenen Schädigungen. Hieraus ergibt sich für die Erfindung die Erkenntnis, daß der unterhalb von etwa 460 nm liegende Anteil der zur Behandlung der Hyperbilirubinämie eingesetzten Strahlung möglichst unterdrückt werden muß.

Auf der anderen Seite ergab sich bei den der Erfindung zugrundeliegenden umfangreichen Untersuchungen, daß Strahlung, die langwelliger als etwa 480 bis 490 nm ist, zu einer Umkehrung der Isomerisation führen kann; die wasserlöslichen Isomere des Bilirubins werden hierbei wieder in fettlösliche umgewandelt. Durch diesen Strahlungsbereich wird daher die erwünschte Wirkung zumindest teilweise wieder aufgehoben. Daraus ergibt sich für die Erfindung die weitere Erkenntnis, daß in der zur phototherapeutischen Behandlung der Hyperbilirubinämie benutzten Strahlung auch die Strahlungsanteile über etwa 480 nm möglichst weitgehend unterdrückt werden sollen.

Erfindungsgemäß liegen daher mindestens 50% der im wirksamen Bereich von 405 bis 545 nm vorhandenen Strahlung zwischen 460 und 480 nm. Zweckmäßig liegen in diesem Bereich von 460 bis 480 nm mindestens 50% der gesamten Strahlung, vorzugsweise jedoch mindestens 90% der gesamten Strahlung.

Die Bestrahlungsstärke innerhalb des Bereiches von 460 bis 480 nm beträgt in der Nutzebene mindestens 2 W/m², vorzugsweise 5 bis 30 W/m².

Weitere wesentliche Einzelheiten der Erfindung betreffen die Art der verwendeten Strahler und Filter.

So kann als Strahler eine mit Leuchtstoff beschichtete Quecksilberdampf-Niederdrucklampe mit hohem Strahlungsanteil zwischen 460 und 480 nm Verwendung finden. Eine solche Quecksilberdampf-Niederdrucklampe ist dabei vorzugsweise mit einem Leuchtstoff beschichtet, der im wesentlichen aus mit Europium aktiviertem Barium-Magnesium-Aluminat und/oder mit Antimon aktiviertem Calcium-Fluor-Phosphat und/oder mit Blei aktiviertem Calcium-Wolframat und/oder mit Zinn aktiviertem Strontium-Pyrosphat und/oder mit Barium-Titan-Pyrophosphat und/oder mit Wolfram aktiviertem Magnesium-Wolframat und/oder mit Antimon und Mangan aktiviertem Strontium-Fluor-Phosphat besteht.

Zur Erhöhung der Strahlungsausbeute kann eine solche Quecksilberdampf-Niederdrucklampe eine innen und/oder außen angebrachte Reflexschicht aufweisen.

Für den erfindungsgemäßen Zweck kann jedoch als Strahler auch eine mit Leuchtstoff beschichtete Quecksilberdampf-Niederdrucklampe Verwendung finden, die mit Amalgam, vorzugsweise Indium-Amalgam, anstelle des elementaren Quecksilbers gefüllt ist und einen hohen Strahlungsanteil zwischen 460 und 480 nm aufweist. Eine solche Lampe kann höher belastet werden.

Gemäß einer anderen Ausgestaltung der Erfindung ist als Strahler ein mit Cadmium-Halogenid und/oder Zink-Halogenid und/oder Thulium-Halogenid und/oder mit Halogeniden anderer seltener Erden dotierter Quecksilberdampf-Hochdruckstrahler vorgesehen. Werden hierbei Cadmium-Halogenid, Zink-Halogenid und/oder Thulium-Halogenid zur Dotierung gewählt, so liegt deren Menge zweckmäßig jeweils zwischen 0,01 und 4 mg/cm³. Als Halogenide werden vorzugsweise Jodide verwendet.

Im Rahmen der Erfindung können auch Hochdruckstrahler Verwendung finden, die keine Quecksilberfüllung enthalten, sondern mit Cadmium-Halogenid, Zink-Halogenid und/oder Thulium-Halogenid gefüllt sind. In diesem Falle liegt der Gehalt an diesen Halogeniden zweckmäßig zwischen 0,02 und 8 mg/cm³. Vorzugsweise sind diese Halogenide Jodide.

Zur Unterdrückung der unterhalb 460 nm liegenden Strahlung sowie gegebenenfalls zur Unterdrückung der oberhalb 480 nm liegenden Strahlung enthält die erfindungsgemäße Vorrichtung wenigstens einen Filter bzw. ein Filtersystem. Es soll in der Nutzebene einen möglichst großen Anteil der vom Strahler emittierten Strahlung im Bereich von 460 bis 480 nm ergeben, dagegen die außerhalb dieses Bereiches liegende Strahlung, insbesondere die Strahlung unterhalb von 460 nm, so weitgehend wie möglich ausfiltern.

Im Rahmen der Erfindung können Absorptionsfilter, Interferenzfilter und geeignete Kombinationen dieser Filtertypen Verwendung finden.

Absorptionsfilter enthalten Zuschlagstoffe, die die unerwünschten Spektralbereiche absorbieren. Finden Glasfilter Verwendung, so wird zur Unterdrückung der unterhalb von 460 nm liegenden Strahlung zweckmäßig ein Anlaufglas (z. B. Schott GG475 3 mm) verwendet, in dem Schwefel- und/oder Cadmiumsulfid gelöst sind; zur Unterdrückung der oberhalb von 480 nm liegenden Strahlung wird ein Absorptionsfilter aus Glas mit Ionenfärbung (z. B. Schott BG37 5 mm) benutzt, in dem Nickeloxyd und/oder Kobaltoxyd gelöst sind.

Gegebenenfalls kann zusätzlich noch ein Wärmeabsorptionsfilter zur Unterdrückung restlicher Infrarotstrahlung vorgesehen werden, um die Belastung der Neugeborenen so gering wie möglich zu halten (z. B. Schott KG1 6 mm). Es können jedoch auch Absorptionsfilter aus Kunststoff benutzt werden. Als Grundstoff findet ein transparentes Kunststoffmaterial Verwendung (polymeres Methylmetacrylat - PMMA, Polycarbonat, Polyester, PVC, Polystyrol etc). Die Kante zum kurzwelligen Bereich (unterhalb von 460 nm) wird hierbei durch einen gelben Farbstoff bewirkt, beispielsweise durch 200 bis 3000 mg/m² Pyrazolon-Derivat. Die Kante zum langwelligen Bereich (oberhalb von 480 nm) wird durch einen blauen Farbstoff hergestellt, beispielsweise durch 200 bis 3000 mg/m² Anthrachinon-Derivat.

Dabei kann es sich entweder um gesonderte Filter handeln, oder es können beide Farbstoffe gemeinsam in das als Folie oder Platte ausgebildete Kunststoffmaterial eingebracht bzw. als Lackschicht auf den Kunststoff aufgebracht werden.

Im Rahmen der Erfindung können ferner auch Interferenzfilter Verwendung finden. Hierbei sind auf ein transparentes Trägermaterial (z. B. aus Quarz, Glas oder Kunststoff) dünne Schichten aufgebracht (meist aufgedampft), die in ihrem Aufbau so abgestimmt sind, daß nur der erwünschte Spektralbereich reflektiert oder transmittiert wird. Bei Interferenz-Transmissionsfiltern können Linien-, Band-, Doppellinien- oder Doppelbandfilter verwendet werden, deren maximale Transmission bei etwa 470 nm liegt.

Interferenzfilter in Form von Reflexionsfiltern sind dichroitische Spiegel, die die auffallende Strahlung mit geringen Verlusten trennen. Dabei kann die Kante zum kurzwelligen Strahlungsbereich durch Veränderung des Einfallswinkels verschoben werden (z. B. Schott 312). Ebenso kann auch die Kante zum langwelligen Bereich durch eine Änderung des Einfallswinkels verschoben werden (z. B. Schott 960). Die beiden letztgenannten Filter transmittieren damit in ihrer Kombination selektiv den erwünschten Spektralbereich von 460 bis 480 nm. Andere dichroitische Spiegel (z. B. Schott 930) reflektieren diesen Bereich selektiv.

Nach dem vorstehend Ausgeführten versteht es sich, daß auch Kombinationen der verschiedenen Filtertypen verwendet werden können, um den erwünschten Strahlungsbereich von 460 bis 480 nm so steilkantig wie möglich auszufiltern.

Für ein einwandfreies Arbeiten der erfindungsgemäßen Bestrahlungsvorrichtung ist auch eine ausreichende Kühlung des Strahlers und der Filter wesentlich.

Erfindungsgemäß wird ein verwendeter Hochdruckstrahler zweckmäßig auf einer Betriebstemperatur zwischen 600 und 1000° C, vorzugsweise zwischen 700 und 800° C, gehalten, während die optimale Betriebstemperatur für eine Niederdrucklampe bei 40 bis 60° C liegt (jeweils an der Kolbenwand).

Ist der Hochdruckstrahler in einem (vorzugsweise aus glanzeloxiertem Aluminium bestehenden) Reflektor angeordnet, so wird dieser Reflektor durch eine Kühleinrichtung zweckmäßig auf einer Betriebstemperatur von 100 bis 130°C gehalten.

Für Absorptionsfilter sollte die Betriebstemperatur etwa 80°C nicht übersteigen. Zweckmäßig ist hierbei eine solche Führung der Kühlluft, daß der zur Unterdrückung der Strahlung unterhalb 460 nm dienende Absorptionsfilter am stärksten gekühlt wird.

Einige Ausführungsbeispiele der Erfindung sind in der Zeichnung veranschaulicht. Es zeigt

Fig. 1 einen schematischen Schnitt durch eine erfindungsgemäße Bestrahlungsvorrichtung mit Hochdruckstrahler und Glasfiltern;

Fig. 2 einen schematischen Schnitt durch ein Ausführungsbeispiel der Erfindung mit Niederdrucklampe und Kunststoff-Filter;

Fig. 3 ein Diagramm der Spektralverteilung der vom Strahler emittierten Strahlung;

Fig. 4 ein Diagramm des spektralen Filter-Transmissionsgrades;

Fig. 5 ein Diagramm der Spektralverteilung der gefilterten Strahlung.

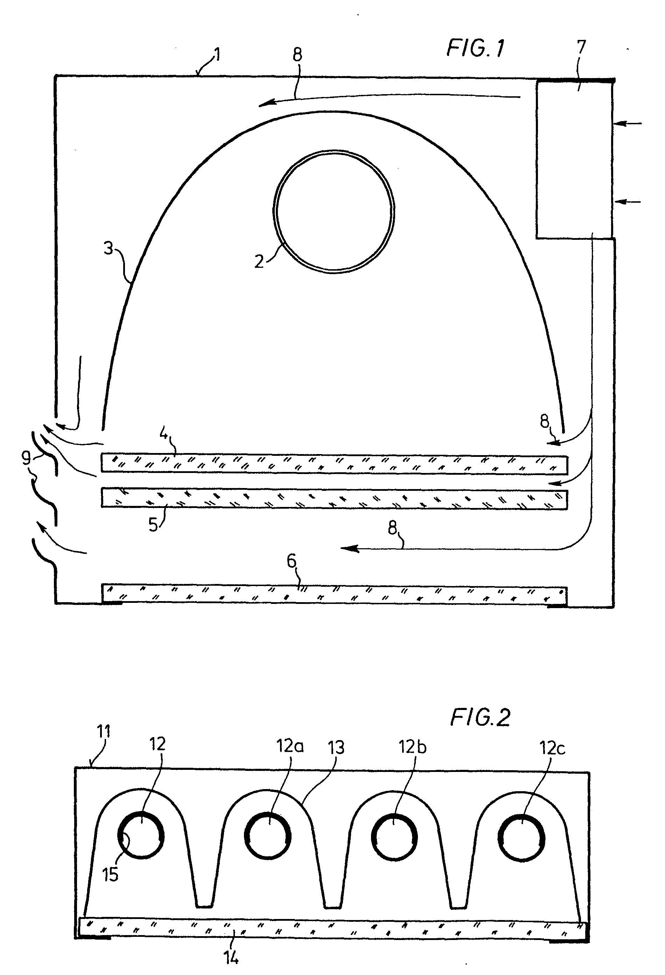

Die in Fig. 1 schematisch dargestellte Bestrahlungsvorrichtung enthält in einem Gehäuse 1 einen Hochdruckstrahler 2, der von einem Reflektor 3 aus glanzeloxiertem Aluminium umgeben ist. Im Strahlengang sind nacheinander Filter 4, 5 und 6 angeordnet.

Der Filter 4 ist ein Wärmeabsorptionsfilter (Schott KG1 6 mm), der Filter 5 ist ein zur Unterdrückung der Strahlung oberhalb 480 nm dienender Blauglasfilter (Schott BG37 5 mm) und der Filter 6 ein die Strahlung unter 460 nm unterdrückender Kantenfilter (Schott GG475 3 mm).

Ein Lüfter 7 fördert Kühlluft längs der dargestellten Pfeile 8 durch das Innere des Gehäuses, insbesondere längs der Filter 4, 5 und 6. Die Kühlluft tritt durch Öffnungen 9 aus dem Gehäuse aus. Der Strahler 2 ist beispielsweise ein Quecksilberdampf-Hochdruckstrahler mit Cadmium-Jodid- und/oder Zink-Jodid-Dotierung. Die Bestrahlungsstärke in der Nutzebene (Abstand 50 cm von dem Filtersystem) beträgt ca. 20 W/m².

Fig. 2 zeigt demgegenüber (in gleichfalls ganz schematischer Form) ein Ausführungsbeispiel der Bestrahlungsvorrichtung, bei dem sich in einem Gehäuse 11 eine Anzahl von Niederdrucklampen 12, 12a, 12b, 12c befindet. Diese Niederdrucklampen sitzen in einem Reflektor 13. Im Strahlengang ist ein Kunststoffilter 14 angeordnet. Der die Niederdrucklampen 12 enthaltende Innenraum des Reflektors 13 ist von Kühlluft durchströmt (die beispielsweise senkrecht zur Zeichenebene der Fig. 2 in Längsrichtung der Niederdrucklampen 12 bis 12c strömt).

Die Niederdrucklampen 12 bis 12c sind beispielsweise Quecksilberdampf-Niederdrucklampen mit Europium aktiviertem Barium-Magnesium-Aluminat-Leuchtstoff und einer aufgebrachten Reflexschicht 15.

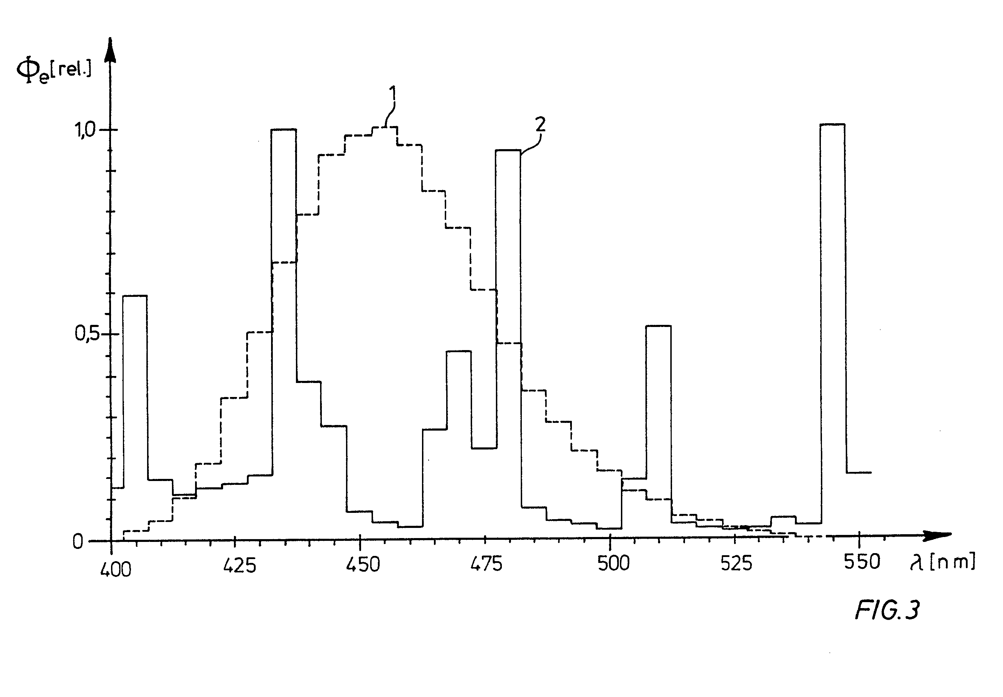

Zur weiteren Erläuterung der Wirkungsweise der beiden Ausführungsbeispiele ist in Fig. 3 der rel. spektr. Strahlungsfluß $O_e$[rel] für die beiden in Fig. 1 und 2 verwendeten Strahler dargestellt: Die Kurve 1 zeigt die spektrale Emission einer mit Europium aktivierten Barium-Magnesium-Aluminat-Leuchtstoff versehene Quecksilberdampf-Niederdrucklampe und Kurve 2 den spektralen Strahlungsfluß eines mit Cadmium-Jodid und Zink-Jodid dotierten Quecksilberdampf-Hochdruckstrahlers.

Man erkennt aus den Kurven gemäß Fig. 3, daß diese beiden Strahler zwar hohe Strahlungsanteile im erwünschten Wellenlängenbereich zwischen 460 und 480 nm besitzen, daß jedoch die außerhalb dieses Bereiches liegenden Strahlungsanteile noch so beträchtlich sind, daß eine Filterung erforderlich ist.

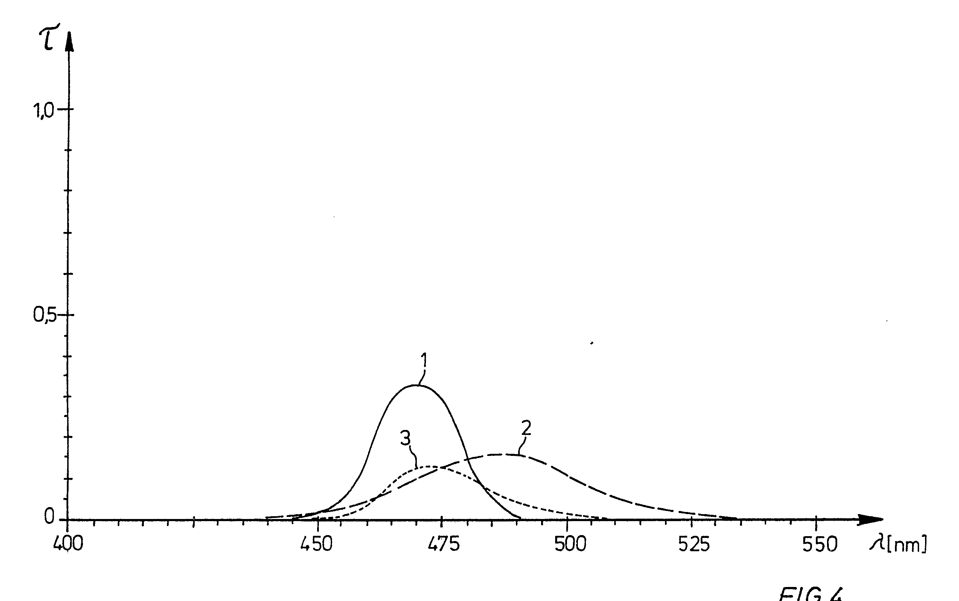

Fig. 4 veranschaulicht den spektralen Transmissionsgrad $\tau$ von drei verschiedenen Filtern: Kurve 1 ist ein Interferenzfilter, Kurve 2 ein Kunststoff-Absorptionsfilter und Kurve 3 ein Glas-Absorptionsfilter.

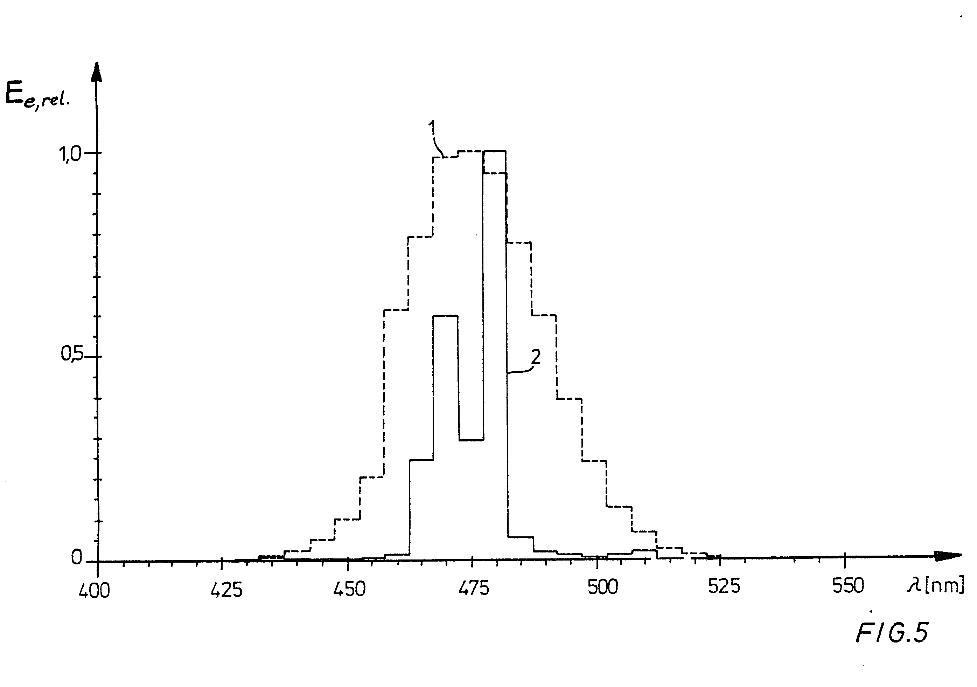

Fig. 5 zeigt schließlich die rel. spektr. Bestrahlungsstärke $E_{e,rel}$ — (Nutzebene) —, wenn man einerseits — Kurve 1 — die Quecksilberdampf-Niederdrucklampe (Kurve 1 gemäß Fig. 3) mit dem Kunststoff-Filter (Kurve 2 gemäß Fig. 4) kombiniert und andererseits — Kurve 2 — den Quecksilberdampf-Hochdruckstrahler (Kurve 2 gemäß Fig. 3) mit einem Glasfilter (Kurve 3 gemäß Fig. 4) kombiniert.

Im ersten Falle (Kurve 1 der Fig. 5) beträgt der zwischen 460 und 480 nm liegende Spektralanteil etwa 60% der gesamten emittierten Strahlung (während bei ungefilterter Strahlung nur etwa 35% der gesamten Strahlung im Bereich zwischen 460 und 480 nm liegen). Die Bestrahlungsstärke im Bereich zwischen 460 und 480 nm beträgt dabei etwa 10 W/m² bei einer Bestrahlungsfläche von 60 · 45 cm und einer Leistungsaufnahme der Strahler von etwa 600 W.

Im zweiten Fall (Kurve 2) beträgt der zwischen 460 und 480 nm liegende Spektralanteil etwa 90% der gesamten emittierten Strahlung. Die Bestrahlungsstärke im Bereich von 460 bis 480 nm liegt bei etwa 20 W/m² bei einer Bestrahlungsfläche von 60 × 45 cm. Die Leistungsaufnahme des Strahlers ist etwa 4000 W.

**Patentansprüche**

1. Vorrichtung zur phototherapeutischen Behandlung der Hyperbilirubinämie von Neugeborenen durch Bestrahlung,
dadurch gekennzeichnet,
daß höchstens 10% der im wirksamen Bereich von 405 bis 545 nm vorhandenen Strahlung unter 460 nm liegen.

2. Vorrichtung nach Anspruch 1, dadurch gekennzeichnet, daß mindestens 50% der im wirksamen Bereich von 405 bis 545 nm vorhandenen Strahlung zwischen 406 und 480 nm liegen.

3. Vorrichtung nach Anspruch 2, dadurch gekennzeichnet, daß mindestens 50% der gesamten Strahlung im Bereich von 460 bis 480 nm liegen.

4. Vorrichtung nach Anspruch 3, dadurch gekennzeichnet, daß mindestens 90% der gesamten Strahlung im Bereich von 460 bis 480 nm liegen.

5. Vorrichtung nach Anspruch 2, dadurch gekennzeichnet, daß die Bestrahlungsstärke innerhalb des Bereiches von 460 bis 480 nm in der Nutzebene mindestens 2 W/m², vorzugsweise 5 bis 30 W/m² beträgt.

6. Vorrichtung nach Anspruch 2, dadurch gekennzeichnet, daß als Strahler eine Quecksilberdampf-Niederdrucklampe mit hohem Strahlungsanteil zwischen 460 und 480 nm vorgesehen ist, die mit einem Leuchtstoff beschichtet ist, der im wesentlichen aus mit Europium aktiviertem Barium-Magnesium-Aluminat und/oder mit Antimon aktiviertem Calcium-Fluor-Phosphat und/oder mit Blei aktiviertem Calcium-Wolframat und/oder mit Zinn aktiviertem Strontium-Pyrophosphat und/oder mit Barium-Titan-Pyrophosphat und/oder mit Wolfram aktiviertem Magnesium-Wolframat und/oder mit Antimon und Mangan aktiviertem Strontium-Fluorphosphat besteht.

7. Vorrichtung nach Anspruch 6, dadurch gekennzeichnet, daß die Quecksilberdampf-Niederdrucklampe einen innen und/oder außen angebrachten Reflexschicht enthält.

8. Vorrichtung nach Anspruch 1, dadurch gekennzeichnet, daß als Strahler eine mit Leuchtstoff beschichtete Quecksilberdampf-Niederdrucklampe mit hohem Strahlungsanteil zwischen 460 und 480 nm vorgesehen ist, bei der Amalgam, vorzugsweise Indium-Amalgam, anstelle von elementarem Quecksilber Verwendung findet.

9. Vorrichtung nach Anspruch 1, dadurch gekennzeichnet, daß als Strahler ein mit Cadmium-Halogenid und/oder Zink-Halogenid und/oder Thulium-Halogenid und/oder mit Halogeniden anderer seltener Erden dotierter Quecksilberdampf-Hochdruckstrahler vorgesehen ist.

10. Vorrichtung nach Anspruch 9, dadurch gekennzeichnet, daß der Quecksilberdampf-Hochdruckstrahler mit je 0,01 bis 4 mg/cm³ Cadmium-Halogenid, Zink-Halogenid und/oder Thulium-Halogenid dotiert ist.

Vorrichtung nach Anspruch 1, dadurch gekennzeichnet, daß als Strahler ein mit Cadmium-Halogenid und/oder Zink-Halogenid und/oder Thulium-Halogenid und/oder mit Halogeniden anderer seltener Erden gefüllter Hochdruckstrahler ohne Quecksilberfüllung vorgesehen ist.

12. Vorrichtung nach Anspruch 11, dadurch gekennzeichnet, daß der Strahler je 0,02 bis 8 mg/cm³ Cadmium-Halogenid, Zink-Halogenid und/oder Thulium-Halogenid enthält.

13. Vorrichtung nach Anspruch 2, dadurch gekennzeichnet, daß zur Unterdrückung der unterhalb 460 nm liegenden Strahlung ein Absorptionsfilter aus Glas mit darin gelöstem Schwefel- und/oder Cadmiumsulfid vorgesehen ist.

14. Vorrichtung nach Anspruch 2, dadurch gekennzeichnet, daß zur Unterdrückung der oberhalb einer vorgegebenen Wellenlänge, vorzugsweise oberhalb 480 nm liegenden Strahlung ein Absorptionsfilter aus Glas mit darin gelöstem Nickeloxyd und/oder Kobaltoxyd vorgesehen ist.

15. Vorrichtung nach Anspruch 2, dadurch gekennzeichnet, daß zusätzlich noch ein Wärmeabsorptionsfilter zur Unterdrückung restlicher Infrarotstrahlung vorgesehen ist.

16. Vorrichtung nach Anspruch 2, dadurch gekennzeichnet, daß zur Unterdrückung der unterhalb 460 nm liegenden Strahlung ein Absorptionsfilter aus Kunststoff mit einem gelben Farbstoff, vorzugsweise 200 bis 3000 mg/m² Pyrazolon-Derivat vorgesehen ist.

17. Vorrichtung nach Anspruch 2, dadurch gekennzeichnet, daß zur Unterdrückung der oberhalb einer vorgegebenen Wellenlänge, vorzugsweise oberhalb 480 nm liegenden Strahlung ein Absorptionsfilter aus Kunststoff mit einem blauen Farbstoff, vorzugsweise 200 bis 3000 mg/m² Anthrachinon-Derivat, vorgesehen ist.

18. Vorrichtung nach Anspruch 9, dadurch gekennzeichnet, daß der Hochdruckstrahler in einem vorzugsweise aus glanzeloxiertem Aluminium bestehenden Reflektor angeordnet ist und eine Kühleinrichtung vorgesehen ist, die den Hochdruckstrahler auf einer Betriebstemperatur zwischen 600 und 1000°C, vorzugsweise zwischen 700 und 800°C, und den Reflektor auf einer Betriebstemperatur zwischen 100 und 130°C hält.

19. Vorrichtung nach den Ansprüchen 13, 14, 16 und 17, dadurch gekennzeichnet, daß eine Kühleinrichtung vorgesehen ist, die die Absorptionsfilter auf einer Betriebstemperatur von maximal etwa 80°C hält.

20. Vorrichtung nach Anspruch 19, gekennzeichnet durch eine solche Führung der von der Kühleinrichtung geförderten Kühlluft, daß der zur Unterdrückung der Strahlung unterhalb 460 nm dienende Absorptionsfilter am stärksten gekühlt wird.

## Claims

1. A phototherapeutic irradiation device for the treatment of hyperbilirubinemia in new born infants characterised in that at most 10% of the radiation present in the effective range of between 405 and 545 nm lies below 460 nm.

2. A device according to claim 1, characterised in that at least 50% of the radiation present in the effective range of between 405 and 545 nm lies between 460 and 480 nm.

3. A device according to claim 2, characterised in that at least 50% of the entire radiation lies in the range between 460 and 480 nm.

4. A device according to claim 3, characterised in that at least 90% of the entire radiation lies in the range between 460 and 480 nm.

5. A device according to claim 1, characterised in that the irradiance within the range between 460 and 480 nm in the irradiated area is at least 2 W/m², preferably between 5 and 30 W/m².

6. A device according to claim 2, characterised in that it comprises as radiator means an low-pressure mercury lamp with a large portion of its radiation lying between 460 and 480 nm, said lamp being coated with a phosphor composed essentially of barium-magnesium aluminate activated with europium and/or calcium-fluorphosphate activated with antimony and/or calcium tungstate activated with lead and/or strontium pyrophosphate activated with tin and/or magnesium tungstate activated with barium-titanium pyrophosphate and/or with tungsten and/or strontium fluorphosphate activated with antimony and manganese.

7. A device according to claim 6, characterised in that the low-pressure mercury lamp has a reflecting layer applied on its interior and/or its exterior.

8. A device according to claim 1, characterised in that it comprises as radiator means a low-pressure mercury lamp coated with a phosphor and having a high portion of its radiation between 460 and 480 nm and in which an amalgam, preferably indium amalgam, is used instead of elementary mercury.

9. A device according to claim 1, characterised in that it comprises as radiator means a high-pressure mercury lamp doped with cadmium halide and/or zinc halide and/or thulium halide and/or halides of other rare earths.

10. A device according to claim 9, characterised in that the high-pressure mercury lamp is doped with 0,01 to 4 mg/cm³ each of cadmium halide, and/or zinc halide, and/or thulium halide.

11. A device according to claim 1, characterised in that it comprises as radiator means a high-pressure lamp doped with cadmium halide and/or zinc halide and/or thulium halide and/or halides or other rare earths, and containing no mercury.

12. A device according to claim 11, characterised in that said radiator means contains 0,02 to 8 mg/cm³ each of the cadmium halide, and/or zinc halide, and/or thulium halide.

13. A device according to claim 2, characterised in that it comprises an absorption filter of glass containing sulfur sulfide and/or cadmium sulfide for the suppression of radiation of wavelengths less than 460 nm.

14. A device according to claim 2, characterised in that it comprises an absorption filter of glass containing dissolved nickel oxide and/or cobalt oxide for the suppression of radiation lying above a predetermined wavelength, preferably radiation above 480 nm.

15. A device according to claim 2, characterised in that it includes an additional heat, absorbing filter for the suppression of residual infrared radiation.

16. A device according to claim 1, characterised in that it comprises an absorption filter of synthetic material with a yellow dye, preferably 200—3000 mg/m² of pyrazolone derivative, for the suppression of radiation below 460 nm.

17. A device according to claim 2, characterised in that it comprises an absorption filter of synthetic material with a blue dye, preferably 200 to 3000 mg/m² of anthraquinone derivate, for the suppression of radiation above a predetermined wavelength, preferably above 480 nm.

18. A device according to claim 1, characterised in that the high-pressure lamp is provided in a revlector, preferably of anode-brightened aluminium, and a cooling system is provided which maintains the high-pressure lamp at an operation temperature between 600 and 1000° C, preferably between 700 and 800° C, and the reflector at an operation temperature between 100 and 130° C.

19. A device according to claims 13, 14, 16 and 17, characterised in that a cooling system is provided which maintains said absorption filters at an operation temperature of about 80° C maximum.

20. A device according to claim 19, characterised in that the flow of cooling air provided by the cooling system is directed such that the best cooling effect is for the absorption filter for suppressing the radiation below 460 nm.

## Revendications

1. Dispositif de traitement photothérapeutique de l'hyperbilirubinémie des nouveaux-nés par rayonnement, caractérisé en ce qu'au maximum 10% du rayonnement situé dans la plage active de 405 à 545 nm sont inférieurs à 460 nm.

2. Dispositif selon la revendication 1, caractérisé en ce qu'au moins 50% du rayonnement situé dans la plage efficace de 405 à 545 nm sont compris entre 460 et 480 nm.

3. Dispositif selon la revendication 2, caractérisé en ce qu'au moins 50% du rayonnement total se trouvent dans la plage de 460 à 480 nm.

4. Dispositif selon la revendication 3, caractérisé en ce qu'au moins 90% du rayonnement total se trouvent dans la plage de 460 à 480 nm.

5. Dispositif selon la revendication 2, caractérisé en ce que l'intensité du rayonnement dans la plage de 460 à 480 nm est d'au moins de 2 W/m², de préférence de 5 à 30 W/m² dans le plan d'utilisation.

6. Dispositif selon la revendication 2, caractérisé en ce que la source de rayonnement est un tube basse pression à vapeur de mercure dont une fraction élevée du rayonnement est comprise entre 460 et 480 nm et qui est enduit d'une substance fluorescente se composant essentiellement d'aluminate de baryum et de magnésium activé à l'europium et/ou de fluophosphate de calcium activé à l'antimoine et/ou de tungsténate de calcium activé au plomb et/ou de pyrophosphate de strontium activé à l'étain et/ou de pyrophosphate de titane et de baryum et/ou de tungsténate de magnésium activé au tungstène et/ou de fluophosphate de strontium activé à l'antimoine et au manganèse.

7. Dispositif selon la revendication 6, caractérisé en ce que le tube basse pression à vapeur de mercure comporte une couche réfléchissante déposée intérieurement et/ou extérieurement.

8. Dispositif selon la revendication 1, caractérisé en ce que la source de rayonnement est un tube basse pression à vapeur de mercure enduit d'une substance fluorescente et dont une fraction élevée du rayonnement est comprise entre 460 et 480 nm, tube dans lequel un amalgame, de préférence un amalgame à l'indium, remplace le mercure élémentaire.

9. Dispositif selon la revendication 1, caractérisé en ce que la source de rayonnement est un tube haute pression à vapeur de mercure dopé par un halogénure de cadmium et/ou un halogénure de zinc et/ou un halogénure de thulium et/ou des halogénures d'autres terres rares.

10. Dispositif selon la revendication 9, caractérisé en ce que la source de rayonnement haute pression à vapeur de mercure est dopée par 0,01 à 4 mg/cm³ d'halogénue de cadmium, d'halogénure de zinc et/ou d'halogénure de thulium.

11. Dispositif selon la revendication 1, caractérisé en ce que la source de rayonnement est un tube haute pression contenant un halogénure de cadmium et/ou un halogénure de zinc et/ou un halogénure de thulium et/ou des halogénures d'autres terres rares et ne renfermant pas de mercure.

12. Dispositif selon la revendication 11, caractérisé en ce que la source de rayonnement contient 0,02 à 8 mg/cm³ d'halogénure de cadmium, d'halogénure de zinc et/ou d'halogénure de thulium.

13. Dispositif selon la revendication 2, caractérisé en ce qu'un filtre d'absorption de verre dans lequel est dissous du sulfure de soufre et/ou du sulfure de cadmium est prévu afin d'arrêter le rayonnement inférieur à 460 nm.

14. Dispositif selon la revendication 2, caractérisé en ce qu'un filtre d'absorption en verre dans lequel est dissous de l'oxyde de nickel et/ou de l'oxyde de cobalt est prévu afin d'arrêter le rayonnement situé au-dessus d'une longueur d'onde prédéterminée, de préférence au-dessus de 480 nm.

15. Dispositif selon la revendication 2, caractérisé en ce qu'il comprend de plus un filtre d'absorption de chaleur destiné à intercepter les restes de rayonnement infrarouge.

16. Dispositif selon la revendication 2, caractérisé en ce qu'un filtre d'absorption de matière plastique comportant un colorant jaune, de préférence 200 à 3000 mg/m² d'un dérivé de la pyrazolone, est prévu pour arrêter le rayonnement inférieur à 460 nm.

17. Dispositif selon la revendication 2, caractérisé en ce qu'un filtre d'absorption de matière plastique comportant un colorant bleu, de préférence 200 à 3000 mg/m² d'un dérivé de l'anthrachinone, est prévu pour arrêter le rayonnement situé au-dessus d'une longueur d'onde prédéterminée, de préférence au- dessus de 480 nm.

18. Dispositif selon la revendication 9, caractérisé en ce que la source de rayonnement haute pression se trouve dans un réflecteur se composant de préférence d'aluminium anodisé brillant et un dispositif de refroidissement est destiné à maintenir la source de rayonnement haute pression à une température de service comprise entre 600 et 1000°C, de préférence entre 700 et 800°C, et le réflecteur, à une température de fonctionnement comprise entre 100 et 130°C.

19. Dispositif selon les revendications 13, 14, 16 et 17, caractérisé en ce qu'un dispositif de refroidissement est prévu afin de maintenir les filtres d'absorption à une température de service qui est au maximum d'environ 80°C.

20. Dispositif selon la revendication 19, caractérisé en ce que l'air de refroidissement refoulé par le dispositif correspondant est conduit de manière à refroidir le plus fortement le filtre d'absorption destiné à arrêter le rayonnement inférieur à 460 nm.

*FIG.1*

*FIG.2*

FIG.3

FIG. 4

FIG.5